Europäisches Patentamt

European Patent Office

Office européen des brevets

(19)

(11) Numéro de publication: **0 276 605**
**B1**

(12)

# FASCICULE DE BREVET EUROPEEN

(45) Date de publication du fascicule du brevet:
**01.08.90**

(21) Numéro de dépôt: **87402924.2**

(22) Date de dépôt: **18.12.87**

(51) Int. Cl.⁵: **C07C 69/63**, C07C 67/11,
C07C 31/40, C07C 29/124

(54) **Synthèse des tetrahydro-1,1,2,2 perfluoroalcanols et de leurs esters.**

(30) Priorité: **22.12.86 FR 8617984**

(43) Date de publication de la demande:
**03.08.88 Bulletin 88/31**

(45) Mention de la délivrance du brevet:
**01.08.90 Bulletin 90/31**

(84) Etats contractants désignés:
**AT BE CH DE ES FR GB IT LI NL SE**

(56) Documents cités:
**EP-A- 0 024 224**
**DE-A- 3 035 641**
**US-A- 3 239 557**

(73) Titulaire: **ATOCHEM, 4 & 8, Cours Michelet La
Défense 10, F-92800 Puteaux(FR)**

(72) Inventeur: **Lantz, André, Domaine de la
Hêtrale 552, Route de Charly, F-6939 Vernaison(FR)**
Inventeur: **Michaud, Pascal, 5 Chemin de la Bastéro Les
Chênes, F-69350 La Mulatiere(FR)**

(74) Mandataire: **Leboulenger, Jean et al, ATOCHEM
Département Propriété Industrielle, F-92091 Paris la
Défense 10 Cédex 42(FR)**

ACTORUM AG

**Description**

La présente invention concerne la préparation des tétrahydro-1,1,2,2 perfluoroalcanols ($R_FCH_2CH_2OH$) et de leurs esters par oxydation des iodures de perfluoroalkyl-2 éthyle correspondants ($R_FCH_2CH_2I$). Le radical perfluoroalkyle $R_F$ peut contenir de 1 à 20 atomes de carbone et être à chaîne linéaire ou ramifiée.

Ces alcools et esters polyfluorés sont des intermédiaires intéressants pour la fabrication d'agents tensioactifs et de produits hydrophobes et oléphobes. Ils peuvent en particulier être facilement transformés en esters acryliques ou méthacryliques dont la polymérisation, éventuellement avec d'autres monomères, fournit des agents d'apprêt hydrophobe et oléphobe pour les matières textiles, le cuir, le papier ou d'autres substrats. Les mélanges d'alcools et d'esters peuvent aussi être transformés entièrement en alcools.

Plusieurs procédés de préparation de ces alcools et esters polyfluorés sont déjà connus. Le procédé décrit dans le brevet FR 1 380 579 qui consiste à faire réagir un iodure $R_FCH_2CH_2I$ avec de l'oléum puis à hydrolyser le sulfate formé, présente l'inconvénient de fournir des quantités importantes de diesters sulfuriques qui sont difficilement hydrolysables.

Selon un procédé décrit dans le brevt US 3 239 557 il est possible d'obtenir les esters $R_FCH_2CH_2OCOR$ par réaction d'un iodure $R_FCH_2CH_2I$ avec un sel d'acide carboxylique RCOOH. Les rendements ne sont cependant pas trés bons car il se forme des quantités plus ou moins importantes d'oléfine $R_FCH=CH_2$.

Les alcools $R_FCH_2CH_2OH$ peuvent aussi être obtenus selon le procédé du brevet FR 2 096 179 qui consiste à préparer les nitrates $R_FCH_2CH_2ONO_2$ par réaction des iodures ($R_FCH_2CH_2I$ avec l'acide nitrique et à hydrogéner ces nitrates en alcool. Ce procédé présente toutefois l'inconvénient de nécessiter deux étapes réactionnelles dont la dernière doit être réalisée sous une pression élevée d'hydrogène.

Le brevet FR 2 180 113 décrit un procédé de fabrication de mélanges d'alcools $R_FCH_2CH_2OH$ et de formiates $R_FCH_2CH_2OCOH$ par réaction à haute température des iodures $R_FCH_2CH_2I$ avec le diméthylformamide en présence d'un peu d'eau. Ce procédé présente l'inconvénient d'exiger des conditions de réaction trés sévères et de fournir comme sous-produit l'oléfine $R_FCH=CH_2$, ce qui diminue d'autant le rendement. Une bonne sélectivité en alcool et formiate ne peut en outre être obtenue qu'en utilisant des quantités trés importantes de diméthylformamide.

Plus récemment, on a proposé dans les brevets EP 0024224 et DE 3 035 641 de préparer ces alcools et esters polyfluorés en faisant réagir l'iodure $R_FCH_2CH_2I$ avec un peracide $RCO_3H$ préalablement formé par l'addition de peroxyde d'hydrogène à un acide carboxylique $RCO_2H$ en présence ou non d'une faible quantité d'acide sulfurique. Cependant, dans les conditions opératoires décrites, ce procédé conduit à une sous-production d'oléfine $R_FCH=CH_2$ en quantité non négligeable et/ou à un faible taux de conversion de l'iodure $R_FCH_2CH_2I$.

Il a maintenant été trouvé un procédé permettant de pallier ces inconvénients, c'est-à-dire d'éviter une sous-production d'oléfine $R_FCH=CH_2$ et d'obtenir néanmoins un excellent taux de conversion.

Le procédé selon l'invention consiste à oxyder un iodure de perfluoroalkyl-2 éthyle au moyen de l'acide mono- ou di- persulfurique dans un acide carboxylique ou un ester d'un tel acide.

L'acide monopersulfurique $H_2SO_5$ et l'acide dipersulfurique $H_2S_2O_8$ peuvent être obtenus suivant différentes méthodes bien connues (P. PASCAL, Nouveau Traité de Chimie Minérale, Tome XIII, 1494-1510). Compte-tenu de son instabilité, l'acide monopersulfurique est de préférence préparé juste avant emploi et ajouté rapidement à une solution du iodure de perfluoroalkyl-2 éthyle dans l'acide ou l'ester carboxylique. L'acide dipersulfurique plus stable peut être utilisé à l'état solide ou sous forme de solution aqueuses.

Comme acides carboxyliques, on utilise de préférence les acides aliphatiques liquides dans les conditions opératoires ; ces acides qui contiennent généralement de 1 à 8 atomes de carbone peuvent être linéaires ou ramifiés, saturés ou non, et contenir des substituants tels que, par exemple, des atomes d'halogène. Conviennent particulièrement bien l'acide acétique propionique.

On peut également utiliser des acides carboxyliques solides tels que les acides aliphatiques lourds ou les acides aromatiques (par exemple l'acide benzoïque et ses dérivés substitués) en ajoutant un solvant tel qu'un alcool (par exemple, méthanol, éthanol, propanol), ou un ester.

Comme indiqué précédemment, on peut utiliser un ester d'acide carboxylique. Cet ester est de préférence un ester d'alcool aliphatique contenant 1 à 4 atomes de carbone, par exemple l'acétate d'éthyle, l'acétate de butyle ou le propionate d'éthyle.

L'oxydation selon l'invention peut être effectuée à une température pouvant aller de -20 à 140°C, mais avantageusement comprise entre 60 et 90°C.

Selon un mode de réalisation préféré de l'invention, on prépare l'acide monopersulfurique par addition de peroxyde d'hydrogène à de l'acide sulfurique entre -5°C et 40°C, puis on ajoute rapidement le mélange à une solution de l'iodure de perfluoroalkyl-2 éthyle dans l'acide ou l'ester carboxylique. Pour une mole de iodure, on met en oeuvre de 1 à 30 moles d'acide sulfurique (de préférence, 5 à 15 moles), de 3 à 20 moles de peroxyde d'hydrogène (de préférence, 5 à 6 moles) et de 1 à 50 moles d'acide ou ester carboxylique (de préférence, 5 à 15 moles).

Le peroxyde d'hydrogène est avantageusement utilisé sous forme de solutions aqueuses dont la concentration en $H_2O_2$ peut varier d'environ 35 à 75 % en poids et est de préférence comprise entre 65 et 75 %.

Bien qu'on préfère utiliser l'acide sulfurique pur ou trés concentré (80 % en poids et plus), on peut également employer des solutions d'acide sulfurique contenant jusqu'à 50 % en poids d'eau.

La réaction d'oxydation est en général très rapide et se fait avec libération d'iode et/ou d'acide iodique qu'on peut séparer facilement du mélange réactionnel par simple filtration. La majeure partie de l'iode peut ainsi être récupérée sous forme d'iode élémentaire par traitement de l'acide iodique avec un réducteur classique tel que le sulfite de sodium.

Les produits fluorés peuvent être isolés suivant les méthodes habituelles, par exemple par décantation et lavage de la phase organique à l'eau. On obtient finalement un produit constitué majoritairement par l'ester de perfluoroalkyl-2 éthyle ($R_FC_2H_4OCOR$) qu'on peut saponifier pour obtenir l'alcool $R_FCH_2CH_2OH$ ou transformer en un autre ester, notamment en acrylate ou méthacrylate.

## AVERTISSEMENT

Comme cela est déjà signalé dans le brevet DE 3.035.641 précité, le travail avec les peracides et le peroxyde d'hydrogène comporte d'importants risques d'inflammation et d'explosion. Il conviendra donc, lors de la mise en oeuvre du procédé selon l'invention, de prendre à cet égard toutes les mesures de précaution usuelles.

Les exemples suivants dans lesquels les pourcentages s'entendent en poids, illustrent l'invention sans la limiter.

## EXEMPLE 1

En vue de préparer l'acide de Caro $H_2SO_5$, on introduit 50 ml d'acide sulfurique à 98 % dans un erlenmeyer plongé dans de la glace, puis on y ajoute goutte à goutte en une heure 24,7 g d'une solution aqueuse de peroxyde d'hydrogène à 70 % (soit 0,5 mole de $H_2O_2$).

Le mélange obtenu est ensuite ajouté rapidement (en 5 à 10 minutes) à une solution de 47,4 g (0,1 mole) d'iodure de perfluorohexyl-2 éthyle $C_6F_{13}C_2H_4I$ dans 60 g d'acide acétique. La température du mélange réactionnel passe rapidement de 20°C à 75-80°C et cette dernière est encore maintenue par chauffage pendant environ 45 minutes.

Après filtration du mélange réactionnel pour éliminer l'acide iodique formé, on décante le filtrat et on lave la phase organique trois fois avec 50 ml d'eau à 25°C. On obtient ainsi 38 g de phase organique dont l'analyse chromatographique montre la répartition suivante des composés fluorés :
94,8 % de $C_6F_{13}C_2H_4OCOCH_3$
1,9 % de $C_6F_{13}C_2H_4OH$
3 % de $(C_6F_{13}C_2H_4O)_2SO_2$
0,3 % de $C_6F_{13}C_2H_4I$ non transformé.

## EXEMPLE 2

En opérant comme à l'exemple 1 à partir de 57,4 g (0,1 mole) d'iodure de perflurooctyl-2 éthyle $C_8F_{17}C_2H_4I$, on obtient 43 g de phase organique dont la répartition des composés fluorés est la suivante :
92 % de $C_8F_{17}C_2H_4OCOCH_3$

1,8 % de $C_8F_{17}C_2H_4OH$
3,1 % de $(C_8F_{17}C_2H_4O)_2SO_2$
3,1 % de $C_8F_{17}C_2H_4I$ non transformé.

## EXEMPLE 3

On opère comme à l'exemple 1, mais en remplaçant l'iodure de perfluorohexyl-2 éthyle par 53,8 g d'un mélange d'iodures $R_FC_2H_4I$ ayant la composition pondérale suivante :

| $R_F$ | % |
|---|---|
| $C_6F_{13}$ | 56,1 |
| $C_8F_{17}$ | 25,0 |
| $C_{10}F_{21}$ | 10,1 |
| $C_{12}F_{25}$ | 4,0 |
| $\geq C_{14}F_{29}$ | 4,8 |

le poids moléculaire moyen de ce mélange étant voisin de 538.

La répartition des composés fluorés dans la phase organique ainsi obtenue (40 g) est la suivante :
80 % de $R_FC_2H_4OCOCH_3$
4,5 % de $R_FC_2H_4OH$
14,7 % de $(R_FC_2H_4O)_2SO_2$
0,8 % de $R_FC_2H_4I$ non transformé.

## EXEMPLE 4

Dans un bécher refroidi par un bain d'eau glacée, on introduit 15 ml d'acide sulfurique à 98 %, puis en environ 15 minutes 10 g de persulfate de potassium. Après dilution du mélange avec un volume égal d'eau, on l'ajoute rapidement à une solution de 4,7 g d'iodure de perfluorohexyl-2 éthyle dans 10 ml d'acide acétique et on laisse réagir pendant 2 heures.

Après décantation, l'analyse chromatographique de la phase organique (4 g) montre la répartition suivante des composés fluorés:
90 % de $C_6F_{13}C_2H_4OCOCH_3$
7 % de $C_6F_{13}C_2H_4OH$
3 % de $C_6F_{13}C_2H_4I$ non transformé.

## Revendications

1. Procédé de préparation des tétrahydro-1,1,2,2 perfluoroalcanols et de leurs esters, caractérisé en ce qu'il consiste à oxyder un iodure de perfluoroalkyl-2 éthyle au moyen de l'acide mono- ou dipersulfurique dans un acide carboxylique ou un ester d'un tel acide.

2. Procédé selon la revendication 1, dans lequel la réaction est effectuée à une température comprise entre 60 et 90°C.

3. Procédé selon la revendication 1 ou 2, dans lequel on prépare l'acide monopersulfurique par addition de peroxyde d'hydrogène à de l'acide sulfurique, puis on ajoute le mélange à une solution d'iodure de perfluoroalkyl-2 éthyle dans l'acide ou l'ester carboxylique.

4. Procédé selon la revendication 3, dans lequel on utilise de 1 à 30 moles d'acide sulfurique, de 3 à 20 moles de peroxyde d'hydrogène et de 1 à 50 moles d'acide ou ester carboxylique par mole d'iodure de perfluoroalkyl-2 éthyle.

**Patentansprüche**

1. Verfahren zur Herstellung von 1,1,2,2-Tetrahydroperfluor-alkanolen und ihren Estern, gekennzeichnet durch die Oxidation von 2-Perfluoralkyl-ethyljodid mittels Mono- oder Diperschwefelsäure in einer Carbonsäure oder ihrem Ester.

2. Verfahren nach Anspruch 1, bei dem die Reaktion bei Temperaturen zwischen 60°C und 90°C durchgeführt wird.

3. Verfahren nach Anspruch 1 oder 2, bei dem man die Monoperschwefelsäure durch Zugabe von Wasserstoffperoxid zur Schwefelsäure herstellt und anschließend das Gemisch zu einer Lösung von 2-Perfluoralkyl-ethyljodid in einer Carbonsäure oder ihrem Ester gibt.

4. Verfahren nach Anspruch 3, bei dem man zwischen 1 und 30 Molen Schwefelsäure, zwischen 3 und 20 Molen Wasserstoffperoxid und zwischen 1 und 50 Molen Carbonsäure oder ihrem Ester pro Mol 2-Perfluoralkyl-ethyljodid verwendet.

**Claims**

1. Process for the preparation of 1,1,2,2-tetrahydroperfluoroalkanols and their esters, characterized in that it consists in oxidizing a 2-perfluoroalkylethyl iodide by means of mono- or di-persulphuric acid in a carboxylic acid or an ester of such an acid.

2. Process according to Claim 1, in which the reaction is carried out at a temperature of between 60 and 90°C.

3. Process according to Claim 1 or 2, in which monopersulphuric acid is prepared by addition of hydrogen peroxide to sulphuric acid and the mixture is then added to a solution of 2-perfluoroalkylethyl iodide in the carboxylic acid or ester.

4. Process according to Claim 3, in which from 1 to 30 moles of sulphuric acid, from 3 to 20 moles of hydrogen peroxide and from 1 to 50 moles of carboxylic acid or ester are used per mole of 2-perfluoroalkylethyl iodide.